# EUROPÄISCHE PATENTSCHRIFT

(11) **EP 1 022 017 B1**
(45) Veröffentlichungstag und Bekanntmachung des Hinweises auf die Patenterteilung: **20.04.2005**
(21) Anmeldenummer: 00100928.1
(22) Anmeldetag: 18.01.2000
(51) Int. Cl.: A61K 7/42, A61K 47/02, A61K 7/48, A61K 47/36

(54) **Kosmetische oder pharmazeutische Zubereitungen für die topische Verabreichung mit verbesserter Stabilität**
Cosmetic or pharmaceutical compositions for topical administration with improved stability
Compositions cosmetiques ou pharmaceutiques pour l'application locale ayant une stabilité ameliorée

(30) Priorität: 20.01.1999 DE 29900938 U; 07.07.1999 DE 19931205
(43) Veröffentlichungstag der Anmeldung: 26.07.2000
(73) Patentinhaber: STADA ARZNEIMITTEL AG, 61118 Bad Vilbel (DE)
(72) Erfinder: Frosch, Vera, 67098 Bad Dürkheim (DE); Hansen, Peter Dr., 63303 Dreieich (DE); Heppner, Andrea, 61197 Florstadt (DE); Schumann, Christof, 35767 Breitscheid-Erdbach (DE)
(74) Vertreter: Hamm, Volker, Dr.

(56) Entgegenhaltungen:
- EP-A- 0 787 483
- EP-A- 0 795 315
- WO-A-87/07139
- FR-A- 2 189 491
- US-A- 3 998 973
- US-A- 5 182 103

## Beschreibung

Die vorliegende Erfindung betrifft kosmetische oder pharmazeutische Zubereitungen für die topische Verabreichung mit verbesserter Stabilität, die Magnesium-Aluminiumsilikat ggf. in Kombination mit Stärke oder Stärkederivaten umfassen gemäß Anspruch 1. Des weiteren betrifft die Erfindung die Verwendung von Magnesium-Aluminiumsilikat ggf. in Kombination mit Stärke oder Stärkederivaten zur Herstellung von kosmetischen oder pharmazeutischen Zubereitungen für die topische Verabreichung gemäß Anspruch 10.

Sowohl kosmetische Zubereitungen, wie Lichtschutzzubereitungen, als auch pharmazeutische Zusammensetzungen, die für die topische Verabreichung vorgesehen sind, werden üblicherweise in Form von Lotionen, Cremes, Salben und dergleichen formuliert. Derartige Formulierungen basieren zumeist auf Emulsionen. Emulsionen sind im weiteren Sinne disperse, mehr oder weniger dickflüssige Zubereitungen, die aus zwei oder mehreren ineinander nicht löslichen Flüssigkeiten bestehen, von denen eine wäßrig ist. Liegen zwei miteinander nicht mischbare Flüssigkeiten vor und handelt es sich um einfache Emulsionssysteme, kann man zwei Typen von Emulsionen unterscheiden: Öl-in-Wasser-Emulsionen (O/W-Emulsionen), bei denen das Öl als innere, offene oder disperse Phase und das Wasser als äußere oder geschlossene Phase vorliegt, und Wasser-in-Öl-Emulsionen (W/O-Emulsionen), bei denen das Wasser als innere, offene oder disperse Phase und das Öl als äußere oder geschlossene Phase vorliegt. Da die eine Emulsion bildenden Flüssigkeiten nicht mischbar sind, neigen Emulsionen grundsätzlich zur Phasentrennung. Dieser Phasentrennung wird durch den Zusatz bestimmter, dem Emulsionstyp entsprechender Emulgatoren entgegengewirkt. Darüber hinaus umfassen Emulsionen oftmals auch viskositätserhöhende Zusätze.

Wenngleich die Verwendung von Emulgatoren und viskositätserhöhenden Mitteln grundsätzlich unbedenklich ist, können diese Emulgatoren im Einzelfall Überempfindlichkeitsreaktionen hervorrufen. Beispielsweise werden im Falle von Lichtschutzzubereitungen Emulsionen und Zusätze zur Einstellung der Viskosität seit längerem im Zusammenhang mit Unverträglichkeitsreaktionen, wie beispielsweise der bekannten Mallorca-Akne". kritisch diskutiert.

Emulgatorfreie Zubereitungen für die topische Verabreichung sind vor allem auf dem Gebiet der Lichtschutzpräparate als sog. Hydrodispersionen verfügbar. Bei Hydrodispersionen handelt es sich grundsätzlich um Dispersionen einer flüssigen, halbfesten oder festen inneren Lipidphase in einer äußeren wäßrigen Phase, die im wesentlichen emulgatorfrei sind. Hydrodispersionen neigen wie Emulsionen zur Phasentrennung.

Eine Stabilisierung von Hydrodispersionssystemen kann dadurch erfolgen, daß in der wäßrigen Phase ein Gelgerüst aufgebaut wird, in dem die Lipidtröpfchen stabil suspendiert sind; man bezeichnet derartige Systeme als Hydrodispersionsgele.

Zur Erzeugung des Gelgerüstes werden bei vorbekannten Hydrodispersionsgelen Konsistenzgeber verwendet, bei denen es sich zumeist um Polyacrylate, beispielsweise aus der Pemulen®- oder Carbopol®-Reihe handelt; siehe beispielsweise DE-OS 195 47 634. Derartige Polyacrylate zeigen jedoch diverse Nachteile, wie beispielsweise den Restlösemittelgehalt einiger Polyacrylattypen, eine hohe Empfindlichkeit gegenüber Salzen, die bei seewasserfesten Formulierungen sowie wegen der auf der Hautoberfläche, zum Beispiel durch starkes Schwitzen befindlichen Salzen nachteilig ist, die Notwendigkeit der pH-Wert-Einstellung (Neutralisation), die oftmals mit Triethanolamin erfolgt, wobei sich bei letzterem wiederum das Problem der Bildung von Nitrosaminen ergibt, die als krebserregend eingestuft werden.

Aufgabe der vorliegenden Erfindung ist es somit, eine kosmetische oder pharmazeutische Zubereitung zur topischen Verabreichung anzugeben, die gegenüber vorbekannten Zubereitungen eine verbesserte Verträglichkeit und eine höhere Stabilität aufweisen.

Diese Aufgabe wird durch die in den unabhängigen Ansprüchen definierten Zusammensetzungen gelöst.

Die abhängigen Ansprüche definieren vorteilhafte Ausführungsformen der Erfindung.

In den der vorliegenden Erfindung zugrundeliegenden Untersuchungen wurde überraschend gefunden, daß unter Verwendung von Magnesium-Aluminiumsilikat stabile Hydrodispersionsgele mit einem sehr guten Hautgefühl und einer hervorragenden Hautverträglichkeit formuliert werden können. Besonders stabile Hydrodispersionsgele werden erfindungsgemäß erhalten, wenn das Magnesium-Aluminisumsilikat in Kombination mit Stärke bzw. einem Stärkederivat eingesetzt wird. Der Vorteil der erfindungsgemäßen Gelsysteme liegt darin, daß zur Gelbildung natürliche Produkte verwendet werden, die nicht mit der von synthetischen Konsistenzgebern wie Polyacrylaten bekannten Restlösemittelproblematik behaftet sind. Erfindungsgemäß lassen sich somit emulgatorfreie, stabile Hydrodispersionsgele bilden. Weitere Vorteile der erfindungsgemäßen Gelsysteme sind daran zu sehen, daß sie nicht salzempfindlich sind und bei ihrer Formulierung auch keinen Zusatz von Neutralisationsmitteln erfordern.

Das erfindungsgemäß verwendete Magnesium-Aluminiumsilikat kann beispielsweise unter dem Warenzeichen Veegum®" von der Vanderbilt Company in Norwalk, USA, erhalten werden kann. Der Gehalt an Magnesium-Aluminiumsilikat in den erfindungsgemäßen Zusammensetzungen beträgt 0,05 bis 20 Gew.-%, bevorzugt 0,1 bis 5 Gew.-% und meist bevorzugt 1 bis 3 Gew.-%.

Gemäß einer bevorzugten Ausführungsform umfassen die erfindungsgemäßen Zubereitungen des weiteren einen Gehalt an Stärke bzw. einem Stärkederivat. Als Stärke kann erfindungsgemäß beispielsweise Maisstärke verwendet werden, als Stärkederivat wird erfindungsgemäß Hydroxypropylstärkephosphat bevorzugt, das von National Starch and Chemical Limited bezogen werden kann. Der Gehalt an Stärke bzw. Stärkederivat in den erfindungsgemäßen Zubereitungen beträgt 0,05 bis 20 Gew.-%, bevorzugt 0,1 bis 5 Gew.-% und meist bevorzugt 1 bis 3 Gew. -%, bezogen auf die Gesamtzusammensetzung.

Die erfindungsgemäßen Zubereitungen umfassen in der Regel eine Ölphase sowie eine wäßrige Phase. Die Ölphase kann dabei aus Fetten, Ölen, gelösten Wachsen oder sonstigen lipophilen Bestandteilen und im Falle der erfindungsgemäßen Lichtschutzzubereitungen auch aus öllöslichen UV-Filtersubstanzen bestehen. Als Öle können vorteilhaft Paraffinöl, mittelkettige Triglyceride, Jojobaöl, Erdnußöl, Kokosöl oder Rizinusöl enthalten sein. Die Öle sind vorzugsweise in einer Menge von 1 bis 10 Gew.-%, bezogen auf die Gesamtzusammensetzung, in den erfindungsgemäßen Zubereitungen enthalten.

Die wäßrige Phase der erfindungsgemäßen Zubereitungen wird aus Wasser, ggf. unter Zusatz von Glycerin, Alkohol, Propylenglykol, Pentylenglykol oder ähnlichen Verbindungen gebildet.

Erfindungsgemäß kann das Magnesium-Aluminiumsilikat, ggf. in Kombination mit Stärke bzw. Stärkederivaten, zur Formulierung kosmetischer Zusammensetzungen, insbesondere Lichtschutzzubereitungen, als auch zur Formulierung von topisch zu verabreichenden pharmazeutischen Zubereitungen verwendet werden. Sie können z. B. als Lotionen, Gele, Cremes, Salben, Stifte und dgl. formuliert werden.

Die unter Verwendung der erfindungsgemäßen Kombination aus Magnesium-Aluminiumsilikat und Stärke bzw. Stärkederivat hergestellten Lichtschutzzubereitungen umfassen mindestens eine UV-Filtersubstanz. Als geeignete, in den erfindungsgemäßen Zusammensetzungen enthaltene organische UV-Filter sind zu nennen:
- Ester der Zimtsäure, beispielsweise p-Methoxyzimtsäureisoamylester (Isoamyl p-Methoxycinnamat) oder Methoxyzimtsäureoctylester (Octyl Methoxycinnamat);
- p-Aminobenzoesäuren, ihre Ester und Derivate, beispielsweise 2-Ethylhexyl-pdimethylaminobenzoat;
- Benzophenone, beispielsweise Benzophenon-3 oder Benzophenon-4;
- Benzylidencampherderivate, beispielsweise 4-Methylbenzylidencampher;
- Dibenzoylmethane, beispielsweise Butylmethoxydibenzoylmethan;
- Octyltriazon
- Octocrylene
- 2-Phenylbezimidazol-5-Sulfonsäure und
- Drometrizole Trisiloxan.

Die genannten UV-Filter können in Mengen zwischen jeweils 1 und 15 Gew.-% in den erfindungsgemäßen Zusammensetzungen enthalten sein. Neben diesen chemischen Filtersubstanzen können die erfindungsgemäßen Zusammensetzungen auch oder zusätzlich physikalische Lichtschutzfilter in Mengen von 1 bis 15 Gew.-% enthalten, wie beispielsweise Titandioxid oder Zinkoxid. Dabei ist es besonders vorteilhaft, wenn die physikalischen Lichtschutzfilter in hydrophober Form vorliegen, d.h. das sie oberflächig wasserabweisend ausgestattet sind. Dies kann zum Beispiel dadurch erfolgen, daß die Pigmentteilchen mit einer hydrophoben Oberflächenschicht aus Silikon überzogen werden. Derartige Pigmente sind kommerziell erhältlich, beispielsweise von der Firma Tayca unter der Handelsbezeichnung MT 100 T.

Die erfindungsgemäßen kosmetischen Zubereitungen umfassen neben ggf. enthaltenen UV-Filtersubstanzen übliche kosmetische Wirkstoffe bzw. Zusatzstoffe, wie pflegende Öle, anfeuchtende und/oder feuchthaltende Substanzen, Fette, Öle, Wachse, Antioxidationsmittel, Konservierungsmittel, Parfüme, Farbstoffe, Stabilisatoren und dergleichen.

Die erfindungsgemäßen pharmazeutischen Zubereitungen für die topische Verabreichung können grundsätzlich alle Wirkstoffe umfassen, die für die topische Verabreichung vorgesehen sind. Als Beispiele für in den erfindungsgemäßen pharmazeutischen Zubereitungen enthaltene Wirkstoffe sind zu nennen:
- Nicht steroidale Antirheumatika:
   z.B. Diclofenac, Piroxicam, Indometazin
- Sonstige Rheumatika:
   z.B. Benzylnicotinat, Hydroxyethylsalicylat
- Virustatika, Antimykotika, Antibiotika:
   z.B. Aciclovir, Clotrimazol, Ketokonazol, Nystatin, Erythromycin, Gentamycin
- Wundheilungsfördernde und pflegende Wirkstoffe:
   z.B. Dexpanthenol, Nachtkerzenöl, Bufexamac, Harnstoff
- Desinfizientien und Keratolytika:
   z.B. Benzoylperoxid, Harnstoff, Salicylsäure, Zinkoxid, PVP Jod, Chlorhexidin
- Psoriatika: z.B. Nachtkerzenöl, Dithranol
- Corticosteroide: z.B. Hydrocortison
- Lokalanästhetika: z.B. Polidocanol, Lidocain
- Hormone: z.B. Testosteron, Estradiol
- Sonstige Wirkstoffe:
   z.B. Heparine, Campher, ätherische Öle, Natriumbituminosulfonat

Von den vorgenannten Wirkstoffen sind jeweils auch deren Salze, Ester und Derivate in den erfindungsgemäßen Zubereitungen einsetzbar.

Die folgenden Beispiele verdeutlichen die Erfindung, ohne sie zu beschränken.

### Beispiele 1 - 6: Zubereitungen ohne Stärke bzw. Stärkederivat

### Beispiel 1

| | |
|---|---|
| Magnesium-Aluminiumsilikat (Veegum®) | 3% |
| Xanthan Gum | 0,4% |
| Disodium EDTA | 0,1% |
| Pentylene Glycol | 5% |
| Butyl Methoxydibenzoylmethane | 2% |
| 4-Methylbenzylidene Camphor | 2% |
| Isoamyl p-Methoxycinnamate | 3% |
| Octyl Methoxycinnamate | 3% |
| Octyl Triazone | 3% |
| Tocopherylacetate | 0,5% |
| Alcohol Denat. | 10% |
| Wasser | 68% |

Die Herstellung dieser Zusammensetzung erfolgt, indem zunächst vier separate Phasen hergestellt werden:
- Phase I:: Magnesium-Aluminiumsilikat und Xanthan Gum werden unter Rühren in Pentylenglycol dispergiert.
- Phase II:: Disodium EDTA wird in Wasser gelöst.
- Phase III:: Die Filtersubstanzen werden bei ca. 75°C ineinander gelöst, dann wird Tocopherylacetat zugegeben.
- Phase IV:: Alkohol

Dann wird Phase II zu Phase I gegeben und das Gemisch 10 Minuten gerührt und homogenisiert. Nach Zugabe der Phase III wird nochmals 5 Minuten gerührt und homogenisiert. Abschließend wird Phase IV zugegeben und nochmals 5 Minuten homogenisiert.

### Beispiel 2

| | |
|---|---|
| Magnesium-Aluminiumsilikat (Veegum®) | 3% |
| Xanthan Gum | 0,4% |
| Disodium EDTA | 0,1% |
| Pentylene Glycol | 5% |
| Butyl Methoxydibenzoylmethane | 2% |
| 4-Methylbenzylidene Camphor | 2% |
| Isoamyl p-Methoxycinnamate | 3% |
| Octyl Methoxycinnamate | 3% |
| Dioctyl Butamido Triazone | 3% |
| Tocopherylacetate | 0,5% |
| Alcohol Denat. | 10% |
| Wasser | 68% |

### Beispiel 3

| | |
|---|---|
| Magnesium-Aluminiumsilikat (Veegum®) | 3% |
| Xanthan Gum | 0,4% |
| Disodium EDTA | 0,1% |
| Pentylene Glycol | 5% |
| Butyl Methoxydibenzoylmethane | 2% |
| 4-Methylbenzylidene Camphor | 2% |
| Isoamyl p-Methoxycinnamate | 3% |
| Octyl Methoxycinnamate | 3% |
| Octocrylene | 3% |
| Tocopherylacetate | 0,5% |
| Alcohol Denat. | 10% |
| Wasser | 68% |

### Beispiel 4

| | |
|---|---|
| Magnesium-Aluminiumsilikat (Veegum®) | 3% |
| Xanthan Gum | 0,4% |
| Disodium EDTA | 0,1% |
| Pentylene Glycol | 5% |
| Butyl Methoxydibenzoylmethane | 2% |
| 4-Methylbenzylidene Camphor | 2% |
| Isoamyl p-Methoxycinnamate | 3% |
| Octyl Methoxycinnamate | 3% |
| Dioctyl Butamido Triazone | 5% |
| Propylene Glycol Dicapylate/Dicaprate | 5% |
| Tocopherylacetate | 0,5% |
| Alcohol Denat. | 10% |
| Wasser | 61% |

### Beispiel 5

| | |
|---|---|
| Magnesium-Aluminiumsilikat (Veegum®) | 3% |
| Xanthan Gum | 0,4% |
| Disodium EDTA | 0,1% |
| Pentylene Glycol | 5% |
| Butyl Methoxydibenzoylmethane | 2% |
| 4-Methylbenzylidene Camphor | 2% |
| Isoamyl p-Methoxycinnamate | 3% |
| Octyl Methoxycinnamate | 3% |
| Octyl Triazone | 5% |
| Phenyl Trimethicone | 5% |
| Tocopherylacetate | 0,5% |
| Alcohol Denat. | 10% |
| Wasser | 61% |

### Beispiel 6

| | |
|---|---|
| Magnesium-Aluminiumsilikat (Veegum®) | 3% |
| Xanthan Gum | 0,4% |
| Disodium EDTA | 0,1% |
| Pentylene Glycol | 5% |
| Butyl Methoxydibenzoylmethane | 2% |
| 4-Methylbenzylidene Camphor | 2% |
| Isoamyl p-Methoxycinnamate | 3% |
| Octyl Methoxycinnamate | 3% |
| Octyl Triazone | 3% |
| Alkyl Benzoate | 5% |
| Tocopherylacetate | 0,5% |
| Alcohol Denat. | 10% |
| Wasser | 63% |

### Beispiele 7 - 18: Zubereitungen mit Stärke bzw. Stärkederivat

### Beispiel 7

| Inhaltsstoff | Menge (Gew.-%) |
|---|---|
| Magnesium-Aluminiumsilikat | 3 |
| Hydroxypropylstärkephosphat | 2 |
| Na-EDTA | 0,1 |
| Pentylenglycol | 5 |
| Alkylbenzoat | 3 |
| Triethylsilyloxy-phenylsilsesquioxan | 2 |
| Octyl Methoxycinnamat | 3 |
| Isoamyl p-Methoxycinnamat | 3 |
| 4-Methylbenzylidencampher | 2 |
| Dioctyl Butamido Triazon | 3 |
| Butylmethoxydibenzoylmethan | 2 |
| Alkohol denat. | 10 |
| Tocopherolacetat | 0,5 |
| Wasser | 61,4 |

### Beispiel 8

| Inhaltsstoff | Menge (Gew.-%) |
|---|---|
| Magnesium-Aluminiumsilikat | 3 |
| Hydroxypropylstärkephosphat | 2 |
| Na-EDTA | 0,1 |
| Glycerin | 4 |
| Buxus Chinensis | 10 |
| Isopropylmyristat | 5 |
| Alkohol denat. | 10 |
| Tocopherolacetat | 0,5 |
| Wasser | 65,4 |

### Beispiel 9

| Inhaltsstoff | Menge (Gew.-%) |
|---|---|
| Magnesium-Aluminiumsilikat | 3 |
| Hydroxypropylstärkephosphat | 2 |
| Na-EDTA | 0,1 |
| Glycerin | 4 |
| Buxus Chinensis | 5 |
| Myritol® 318 (Caprylic/Capric Triglyceride) | 5 |
| Isopropylmyristat | 5 |
| Butylmethoxydibenzoylmethan | 1 |
| Alkohol denat. | 10 |
| Tocopherolacetat | 1 |
| Wasser | 63,9 |

### Beispiel 10

| Inhaltsstoff | Menge (Gew.-%) |
|---|---|
| Magnesium-Aluminiumsilikat | 3 |
| Hydroxypropylstärkephosphat | 2 |
| Na-EDTA | 0,1 |
| Pentylenglycol | 2 |
| Glycerin | 3 |
| Alkylbenzoat | 5 |
| Myritol® 318 (Caprylic/Capric Triglyceride) | 5 |
| Isopropylmyristat | 5 |
| Octyl Methoxycinnamat | 5 |
| 4-Methylbenzoyliencampher | 2 |
| Octyltriazon | 5 |
| Butylmethoxydibenzoylmethan | 1 |
| Alkohol denat. | 10 |
| Tocopherolacetat | 0,5 |
| Wasser | 51,4 |

### Beispiel 11

| Inhaltsstoff | Menge (Gew.-%) |
|---|---|
| Magnesium-Aluminiumsilikat | 3 |
| Hydroxypropylstärkephosphat | 2 |
| Na-EDTA | 0,1 |
| Pentylenglycol | 5 |
| Glycerin | 4 |
| Buxus Chinensis | 5 |
| Myritol® 318 (Caprylic/Capric Triglyceride) | 5 |
| Isopropylmyristat | 5 |
| Alkohol denat. | 10 |
| Tocopherolacetat | 5 |
| Wasser | 55,9 |

### Beispiel 12

| Inhaltsstoff | Menge (Gew. -%) |
|---|---|
| Magnesium-Aluminiumsilikat | 3 |
| Stärke | 2 |
| Na-EDTA | 0,1 |
| Glycerin | 4 |
| Buxus Chinensis | 10 |
| Isopropylmyristat | 5 |
| Alkohol denat. | 10 |
| Tocopherolacetat | 0,5 |
| Wasser | 65,4 |

### Beispiel 13

| Inhaltsstoff | Menge (Gew.-%) |
|---|---|
| Magnesium-Aluminiumsilikat | 4 |
| Hydroxypropylstärkephosphat | 3 |
| Mittelkettige Partialglyceride | 20 |
| Glycerol | 5 |
| Methyl-4-hydroxybenzoat | 0,1 |
| Propyl-4-hydroxybenzoat | 0,04 |
| Na-EDTA | 0,01 |
| Wasser | 86,25 |
| Diclofenac-Natrium | 1,0 |

### Beispiel 14

| Inhaltsstoff | Menge (Gew.-%) |
|---|---|
| Magnesium-Aluminiumsilikat | 3 |
| Hydroxypropylstärkephosphat | 2,5 |
| Mittelkettige Partialglyceride | 15 |
| Siliconöl | 10 |
| Propylenglycol | 7,5 |
| Ethanol | 10 |
| Wasser | 49,5 |
| Polidocanol | 2,5 |

### Beispiel 15

| Inhaltsstoff | Menge (Gew.-%) |
|---|---|
| Magnesium-Aluminiumsilikat | 3 |
| Hydroxypropylstärkephosphat | 2,5 |
| Mittelkettige Partialglyceride | 15 |
| Isopropylmyristat | 2,5 |
| Propylenglycol | 10 |
| Sorbinsäure | 0,25 |
| Puffer | 0,25 |
| Na-EDTA | 0,01 |
| Wasser | 63,49 |
| Hydrocortisonacetat | 0,25 |

### Beispiel 16

| Inhaltsstoff | Menge (Gew.-%) |
|---|---|
| Magnesium-Aluminiumsilikat | 4 |
| Maisstärke | 4 |
| Mittelkettige Partialglyceride | 25 |
| Isopropylmyristat | 2,5 |
| Siliconöl | 5 |
| Glycerol | 5 |
| Methyl-4-hydroxybenzoat | 0,1 |
| Propyl-4-hydroxybenzoat | 0,04 |
| Puffer | 0,2 |
| Wasser | 53,16 |
| Clotrimazol | 1 |

### Beispiel 17

| Inhaltsstoff | Menge (Gew.-%) |
|---|---|
| Magnesium-Aluminiumsilikat | 3 |
| Hydroxypropylstärkephosphat | 2 |
| Mittelkettige Partialglyceride | 20 |
| Isopropylmyristat | 3 |
| Propylenglycol | 10 |
| Ethanol | 10 |
| Wasser | 47 |
| Dexpanthenol | 5 |

### Beispiel 18

Die Herstellung der erfindungsgemäßen Zubereitungen umfassend eine Kombination aus Magnesium-Aluminiumsilikat und Stärke /Stärkederivat kann auf folgendem Weg erfolgen:

Zunächst werden die Stärke bzw. das Stärkederivat und das Magnesium-Aluminiumsilikat in der Ölphase dispergiert. Parallel werden die Bestandteile der Wasserphase in Wasser gelöst und separat die Bestandteile der Ölphase miteinander vermischt. Anschließend wird die Wasserphase zu der Dispersion aus Stärke bzw. Stärkederivat und Magnesium-Aluminiumsilikat gegeben und 10 Minuten homogenisiert. Unter Rühren wird dann die Ölphase und ggf. die gelösten Lichtschutzfilter zugegeben. Nach erneutem Homogenisieren kann ggf. eine Lösung von Tocopherolacetat in Alkohol zugesetzt werden, wonach erneut homogenisiert wird.

### Beispiel 19

Die Hautverträglichkeit der Sonnenschutzformulierung gemäß Beispiel 7 wurde an sechs freiwilligen Probanden untersucht. Hierzu wurden die Probanden mit dem gefilterten Solar Simulator Sol 5 der Firma Hönle mit 100% UVA-Licht bestrahlt. Die Probanden wurden an fünf aufeinanderfolgenden Tagen je nach Lichtempfindlichkeit, die zuvor in einer einer Lichttreppe ermittelt wurde, mit UVA-Licht bestrahlt. Die Bestrahlungszeit wurde individuell festgelegt und lag unterhalb der Erythemschwelle. Als Positivkontrolle diente eine Testemulsion Zie 12/82-4, unter deren Verwendung durch Bestrahlung mit UVA-Licht eine Mallorca-Akne bei entsprechend disponierten Probanden ausgelöst werden kann.

Die Prüfpräparate wurden 10 min. vor der Bestrahlung auf die Testareale im Dekollete-Bereich aufgetragen, ebenso die Referenzsubstanz. Abgelesen wurden die Reaktionen jeweils direkt nach der Bestrahlung und nochmals nach 24h, also vor einer erneuten Bestrahlung. Das oben beschriebene Verfahren wurde an jeweils fünf aufeinanderfolgenden Tagen wiederholt.

Es zeigte sich bei allen Probanden im Testfeld der Positiv-Kontrolle unter Belichtung mit 100% UVA-Licht eine Hautreaktion im Sinne einer Mallorca-Akne. Dieser Test ist notwendig, um zu gewährleisten, daß es sich bei den Probanden um Personen handelt, bei denen eine Mallorca-Akne unter Laborbedingungen ausgelöst werden kann.

Die erfindungsgemäße Lichtschutzzubereitung zeigte bei keinem der sechs teilnehmenden Probanden Hautreaktionen. Somit kann davon ausgegangen werden, daß sich dieses Produkt eignet, um Hautreaktionen einer Mallorca-Akne zu vermeiden.

## Patentansprüche

1. Emulgatorfreie kosmetische oder pharmazeutische Zubereitung für die topische Verabreichung in Form eines Hydrodispersionsgels,
**dadurch gekennzeichnet, dass** sie einen Gehalt an Magnesium-Aluminiumsilikat umfasst.

2. Zubereitung nach Anspruch 1,
**dadurch gekennzeichnet, dass** das Magnesium-Aluminiumsilikat in einer Menge von 0,1 bis 20 Gew.-%, bezogen auf die Gesamtzusammensetzung, enthalten ist.

3. Zubereitung nach Anspruch 1 oder 2,
**dadurch gekennzeichnet, dass** sie zusätzlich einen Gehalt an Stärke und/oder einem Stärkederivat umfasst.

4. Zubereitung nach Anspruch 3,
**dadurch gekennzeichnet, daß** das Stärkederivat Hydroxypropylstärkephosphat ist.

5. Zubereitung nach Anspruch 4,
**dadurch gekennzeichnet, daß** die Stärke und/oder das Stärkederivat in einer Menge von 0,1 bis 20 Gew.-%, bezogen auf die Gesamtzusammensetzung, enthalten sind.

6. Zubereitung nach einem der Ansprüche 1 bis 5,
**dadurch gekennzeichnet, daß** sie zusätzlich mindestens eine öllösliche und/oder eine wasserlösliche UV-Filtersubstanz umfasst.

7. Zubereitung nach einem der Ansprüche 1 bis 6,
**dadurch gekennzeichnet, daß** sie zusätzlich eine anorganische UV-Filtersubstanz umfasst.

8. Zubereitung nach einem der Ansprüche 1 bis 7,
**dadurch gekennzeichnet, daß** zusätzlich mindestens ein pharmazeutischer Wirkstoff enthalten ist.

9. Zubereitung nach Anspruch 8,
**dadurch gekennzeichnet, daß** der mindestens eine Wirkstoff ausgewählt ist aus der Gruppe bestehend aus nicht-steroidalen Antirheumatika, Rheumatika, Virustatika, Antimykotika, Antibiotika, Wundheilungsfördernde und pflegende Wirkstoffe, Desinfizientien, Keratolytika, Psoriatika, Corticosteroide, Lokalanästhetika und Hormone sowie deren Derivaten.

10. Verwendung von Magnesium-Aluminiumsilikat ggf. in Kombination mit Stärke und/oder Stärkederviaten zur Herstellung emulgatorfreier kosmetischer oder pharmazeutischer Zubereitungen in Form von Hydrodispersionsgelen für die topische Verabreichung.

## Claims

1. Emulsifier-free cosmetic or pharmaceutical preparation for topical application in the form of a hydrodispersion gel,
**characterised in that** it includes a content of magnesium aluminium silicate.

2. Preparation according to claim 1,
**characterised in that** the magnesium aluminium silicate is included in a quantity of 0.1 to 20 wt.%, relative to the overall composition.

3. Preparation according to claim 1 or 2,
**characterised in that** it additionally includes a content of starch and/or a starch derivative.

4. Preparation according to claim 3,
**characterised in that** the starch derivative is hydroxypropyl starch phosphate.

5. Preparation according to claim 4,
**characterised in that** the starch and/or starch derivative are included in a quantity of 0.1 to 20 wt.%, relative to the overall composition.

6. Preparation according to one of claims 1 to 5,
**characterised in that** it additionally includes at least one oil-soluble and/or water-soluble UV filter substance.

7. Preparation according to one of claims 1 to 6,
**characterised in that** it additionally includes an inorganic UV filter substance.

8. Preparation according to one of claims 1 to 7,
**characterised in that** at least one pharmaceutical active ingredient is additionally included.

9. Preparation according to claim 8,
**characterised in that** the at least one active ingredient is selected from the group comprising non-steroidal antirheumatics, rheumatics, virustatics, antimycotics, antibiotics, wound healing promoting and therapeutic agents, disinfectants, keratolytics, psoriatics, corticosteroids, local anaesthetics and hormones and derivatives thereof.

10. Use of magnesium aluminium silicate optionally in combination with starch and/or starch derivatives for the production of emulsifier-free cosmetic or pharmaceutical preparations for topical application in the form of hydrodispersion gels.

## Revendications

1. Préparation cosmétique ou pharmaceutique sans émulsifiant pour administration topique sous forme d'un gel hydrodispersible, **caractérisée en ce qu'**elle comprend une teneur en silicate de magnésium et d'aluminium.

2. Préparation selon la revendication 1, **caractérisée en ce que** le silicate de magnésium et d'aluminium est contenu dans une quantité allant de 0,1 à 20 % en poids, basé sur la composition totale.

3. Préparation selon la revendication 1 ou 2, **caractérisée en ce qu'**elle comprend de plus une teneur en amidon et/ou en un dérivé de l'amidon.

4. Préparation selon la revendication 3, **caractérisée en ce que** le dérivé de l'amidon est le phosphate d'hydroxypropylamidon.

5. Préparation selon la revendication 4, **caractérisée en ce que** l'amidon et/ou le dérivé de l'amidon est/sont contenu(s) dans une quantité allant de 0,1 à 20 % en poids, basé sur la composition totale.

6. Préparation selon l'une des revendications 1 à 5, **caractérisée en ce qu'**elle comprend en outre au moins une substance filtrant les UV soluble dans l'huile et/ou hydrosoluble.

7. Préparation selon l'une des revendications 1 à 6, **caractérisée en ce qu'**elle comprend, de plus, une substance inorganique filtrant les UV.

8. Préparation selon l'une des revendications 1 à 7, **caractérisée en ce qu'**elle comporte en outre au moins une substance active pharmaceutique.

9. Préparation selon la revendication 8, **caractérisée en ce qu'**au moins une substance active est choisie dans le groupe composé d'anti-rhumatoïdes non stéroïdiens, de rhumatoïdes, de virustatiques, d'antimycotiques, d'antibiotiques, de substances actives cicatrisantes et traitantes, de désinfectants, de kératolytiques, d'antipsoriasiques, de corticostéroïdes, d'anesthésiques locaux et d'hormones ainsi que de leurs dérivés.

10. Utilisation de silicate de magnésium et d'aluminium, le cas échéant combiné à de l'amidon et/ou des dérivés de l'amidon, pour l'élaboration de préparations cosmétiques ou pharmaceutiques sans émulsifiant, sous forme d'un gel hydrodispersible pour administration topique.
